# EUROPEAN PATENT SPECIFICATION

(11) **EP 2 797 535 B1**
(45) Date of publication and mention of the grant of the patent: **21.10.2020**
(21) Application number: 12806313.8
(22) Date of filing: 13.12.2012
(51) Int. Cl.: A61B 18/14

(54) **DEVICE FOR RENAL NERVE MODULATION MONITORING**
VORRICHTUNG ZUR ÜBERWACHUNG VON NIERENNERVENMODULATION
DISPOSITIF POUR LA SURVEILLANCE DE LA MODULATION DE NERFS RÉNAUX

(30) Priority: 29.12.2011 US 201161581377 P
(43) Date of publication of application: 05.11.2014
(73) Proprietor: Boston Scientific Scimed, Inc., Maple Grove, MN 55311 (US)
(72) Inventor: SMITH, Scott R., Chaska, Minnesota 55318 (US)
(74) Representative: Vossius & Partner Patentanwälte Rechtsanwälte mbB
(86) International application number: PCT/US2012/069475
(87) International publication number: WO 2013/101485

(56) References cited:
- EP-A1- 2 779 929
- EP-A2- 1 145 686
- WO-A1-2013/074813
- US-A1- 2009 076 409
- US-A1- 2009 182 318
- US-A1- 2011 257 562

## Description

### Technical Field

The present invention relates to apparatuses for nerve modulation techniques such as ablation of nerve tissue or other destructive modulation technique through the walls of blood vessels and monitoring thereof.

### Background

Certain treatments require the temporary or permanent interruption or modification of select nerve function. One example treatment is renal nerve ablation which is sometimes used to treat hypertension and other conditions related to hypertension and congestive heart failure. The kidneys produce a sympathetic response to congestive heart failure, which, among other effects, increases the undesired retention of water and/or sodium. Ablating some of the nerves running to the kidneys may reduce or eliminate this sympathetic function, which may provide a corresponding reduction in the associated undesired symptoms.
Many nerves (and nervous tissue such as brain tissue), including renal nerves, run along the walls of or in close proximity to blood vessels and thus can be accessed intravascularly through the walls of the blood vessels. In some instances, it may be desirable to ablate perivascular renal nerves using a radio frequency (RF) electrode in an off-wall configuration or in a configuration in contact with the vessel wall. RF electrodes may ablate the perivascular nerves, but may also damage the vessel wall as well. Control of the ablation may effective ablate the nerves while minimizing injury to the vessel wall. Sensing electrodes may allow the use of impedance measuring to monitor tissue changes. It is therefore desirable to provide for alternative systems and methods for intravascular nerve modulation.

### Summary

Accordingly, one illustrative embodiment is a system for nerve modulation in accordance with appended claim 1.

### Brief Description of the Drawings

The invention may be more completely understood in consideration of the following detailed description of various embodiments in connection with the accompanying drawings, in which:
Figure 1 is a schematic view illustrating a renal nerve modulation system in situ.
Figure 2 illustrates a distal end of an illustrative renal nerve modulation system.
Figure 3 illustrates a schematic of the electrical circuit.
Figure 4 illustrates a distal end of another illustrative renal nerve modulation system.

While the invention is amenable to various modifications and alternative forms, specifics thereof have been shown by way of example in the drawings and will be described in detail. It should be understood, however, that the intention is not to limit aspects of the invention to the particular embodiments described. On the contrary, the intention is to cover all modifications, equivalents, and alternatives falling within the spirit and scope of the invention.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

Although some suitable dimensions, ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

While the devices and methods described herein are discussed relative to renal nerve modulation, it is contemplated that the devices and methods may be used in other applications where nerve modulation and/or ablation are desired. For example, the devices and methods described herein may also be used for prostate ablation, tumor ablation, and/or other therapies requiring heating or ablation of target tissue. In some instances, it may be desirable to ablate perivascular renal nerves with deep target tissue heating. As energy passes from a modulation element to the desired treatment region the energy may heat both the tissue and the intervening fluid (e.g. blood) as it passes. As more energy is used, higher temperatures in the desired treatment region may be achieved thus resulting in a deeper lesion. Monitoring tissue properties may, for example, verify effective ablation, improve safety, and optimize treatment time.

In some instances, impedance monitoring may be used to detect changes in target tissues as ablation progresses. Sensing electrodes may be provided in addition to the modulation element. In some instances, the impedance may not be directly measured, but may be a function of the current distribution between the sensing electrodes. In general, the resistance of the surrounding tissue may decrease as the temperature of the tissue increases until a point where the tissue begins to denature or irreversibly change, for example, at approximately 50-60°C. Once the tissue has begun to denature the resistance of the tissue may increase. As the target tissue is ablated, the change in impedance may be analyzed to determine how much tissue has been ablated. The power level and duration of the ablation may be adjusted accordingly based on the impedance of the tissue. In some instances, overall circuit impedance may be monitored and modulation systems may utilize a standard power delivery level, but variation in local tissue impedance can cause unpredictable variation in the ablation effect on the target tissue and in local artery wall heating. It may be desirable to provide a simple way to determine local tissue impedance in order to control ablation using a split electrode.

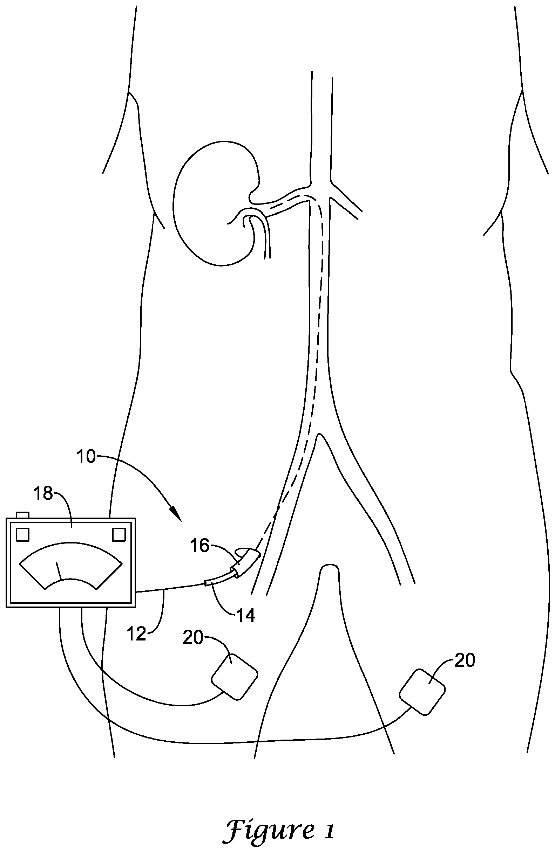

Figure 1 is a schematic view of an illustrative renal nerve modulation system 10 in situ. System 10 may include an element 12 for providing power to a nerve modulation element disposed about and/or within a central elongate shaft 14 and, optionally, within a sheath 16, the details of which can be better seen in subsequent figures. A proximal end of element 12 may be connected to a control and power unit 18, which supplies the necessary electrical energy to activate the one or more modulation elements at or near a distal end of the element 12. In some instances, return electrode patches 20 may be supplied on the legs or at another conventional location on the patient's body to complete the circuit. The control and control unit 18 may include monitoring elements to monitor parameters such as power, temperature, voltage, pulse size, and/or shape and other suitable parameters as well as suitable controls for performing the desired procedure. In some instances, the power unit 18 may control a radio frequency (RF) ablation electrode and/or one or more sensing electrodes. It is contemplated that more than one control unit 18 may be provided. In some instances, one or more sensing and/or ablation electrodes may be connected to separate control units 18. The electrodes are configured to function as both an ablation electrode and a sensing electrode. The ablation electrode may be configured to operate at a frequency of approximately 460 kHz. It is contemplated that any desired frequency in the RF range may be used, for example, from 100 - 500 kHz. However, it is contemplated that different types of energy outside the RF spectrum may be used as desired, for example, but not limited to ultrasound, microwave, and laser to perform the ablation. While the term ablation electrode is used herein, it is contemplated that the modulation element and modulation frequency may be selected according to the energy used to perform the ablation. For example, when ultrasound energy is used, an ultrasonic transducer may be selected as the modulation element and modulation frequencies may be in the MHz range. The sensing electrodes may be configured to operate over frequency ranges which are different from the frequency range at which the ablation is being performed. It is contemplated that the sensing electrodes may be operated over a range of frequencies for improved impedance measuring.

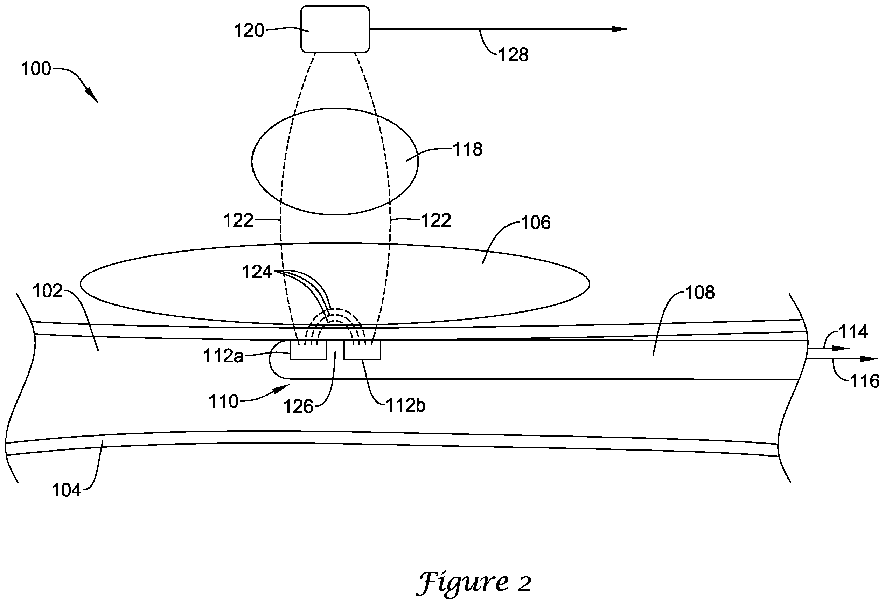

Figure 2 is an illustrative embodiment of a distal end of a renal nerve modulation system 100 disposed within a body lumen 102 having a vessel wall 104. The vessel wall 104 may be surrounded by local body tissue 106. The local body tissue may comprise adventitia and connective tissues, nerves, fat, fluid, etc. in addition to the muscular vessel wall 104. A portion of the tissue 106 may be the desired treatment region. The system 100 may include an elongate shaft 108 having a distal end region 110. The elongate shaft 108 may extend proximally from the distal end region 110 to a proximal end configured to remain outside of a patient's body. The proximal end of the elongate shaft 108 may include a hub attached thereto for connecting other treatment devices or providing a port for facilitating other treatments. It is contemplated that the stiffness of the elongate shaft 108 may be modified to form a modulation system 100 for use in various vessel diameters and various locations within the vascular tree. The elongate shaft 108 may further include one or more lumens extending therethrough. For example, the elongate shaft 108 may include a guidewire lumen and/or one or more auxiliary lumens. The lumens may be configured in any way known in the art. For example, the guidewire lumen may extend the entire length of the elongate shaft 108 such as in an over-the-wire catheter or may extend only along a distal portion of the elongate shaft 108 such as in a single operator exchange (SOE) catheter. These examples are not intended to be limiting, but rather examples of some possible configurations. While not explicitly shown, the modulation system 100 may further include temperature sensors/wire, an infusion lumen, radiopaque marker bands, fixed guidewire tip, a guidewire lumen, external sheath and/or other components to facilitate the use and advancement of the system 100 within the vasculature.

The system 100 may further include a split plate electrode having a first plate 112a and a second plate 112b. A split plate electrode may be two electrodes positioned adjacent to one another. While the term split plate is used to describe the ablation electrodes 112a,b it is to be understood that the electrodes are not limited to a plate structure. For example, the electrodes 112a,b may include a hemispherical tip electrode adjacent the distal end of the elongate shaft 108, wire wrapped coils, generally solid shapes, ball-type electrodes, etc. The electrodes may be connected separately to a control unit (such as control unit 18 shown in Figure 1) but may be operated as a single electrode. The first and second plates 112a,b may be positioned on the elongate shaft 108 in close proximity to each other, as will be discussed in more detail below. While the first and second plates 112a,b may be referred to as ablation electrodes, in some instances, the split plate electrode 112a,b may simultaneously function as both ablation and sensing electrodes as will be described in more detail below. The ablation electrodes 112a,b may be disposed on the outer surface of the elongate shaft 108 adjacent the distal end region 110. However, the ablation electrodes 112a,b may be placed at any longitudinal location along the elongate shaft 108 desired. While the system 100 is illustrated as including one pair of ablation electrodes 112a,b, it is contemplated that the modulation system 100 may include any number of split electrodes 112a,b, such as, but not limited to, two, three, four, or more. If multiple split electrodes 112a,b are provided, the split electrodes 112a,b may be longitudinally, radially and/or circumferentially spaced as desired.

In some embodiments, the ablation electrodes 112a,b may be formed of a separate structure and attached to the elongate shaft 108. For example, the ablation electrodes 112a,b may be machined or stamped from a monolithic piece of material and subsequently bonded or otherwise attached to the elongate shaft 108. In other embodiments, the ablation electrodes 112a,b may be formed directly on the surface of the elongate shaft 108. For example, the ablation electrodes 112a,b may be plated, printed, or otherwise deposited on the surface. In some instances, the ablation electrodes 112a,b may be a radiopaque marker band. The ablation electrodes 112a,b may be formed from any suitable material such as, but not limited to, platinum, gold, stainless steel, cobalt alloys, or other non-oxidizing materials. In some instances, titanium, tantalum, or tungsten may be used. It is contemplated that the ablation electrodes 112a,b may take any shape desired, such as, but not limited to, square, rectangular, circular, elliptical, etc. In some embodiments, the ablation electrodes 112a,b may have rounded edges in order to reduce the affects of sharp edges on current density. The size of the ablation electrodes 112a,b may be chosen to optimize the current density without increasing the profile of the modulation system 100. For example, an ablation electrode 112a,b that is too small may generate high local current densities resulting in greater heat transfer to the blood and surrounding tissues. An ablation electrode 112a,b that is too large may require a larger elongate shaft 108 to carry it. In some instances, the ablation electrodes 112a,b may have an aspect ratio of 2:1 (length to width) or greater. Such an elongated structure may provide the ablation electrodes 112a,b with more surface area without increasing the profile of the modulation system 100.

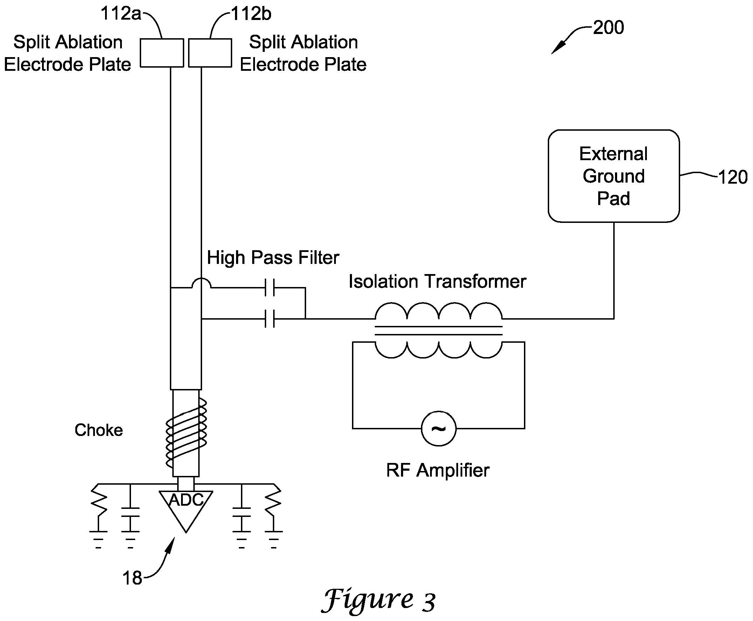

The split electrodes 112a,b may be energized at a first frequency (for example, approximately 500 kHz) to impart tissue change in the surrounding local tissue 106 and a second frequency (for example, approximately 10 MHz) to monitor and detect tissue changes in the local tissue 106. In some embodiments, the split ablation electrodes 112a,b may be connected to a control unit (such as control unit 18 in Figure 1) by separate independent conductors 114, 116. However, it is contemplated that the split electrode plates 112a,b may function as a single electrode. A skin-contact ground pad 120 may also be connected through an electrical conductor 128 to a control unit. In operation, RF ablation energy (such as a voltage waveform) may be applied to both electrode plates 112a,b such that ablation current 122 passes through the local target tissue 106 and additional body tissue 118 to a ground pad 120. It is contemplated that in some instances, current control may be used in addition to or in place of voltage control to control the ablation. In some instances, a higher-frequency signal may be superimposed on the ablation waveform, with the two electrode plates 112a,b acting as positive and negative poles for the high-frequency signal. This may result in a small high-frequency current 124 between the split electrode plates 112a,b. It is contemplated that a choke circuit, such as choke circuit 200 illustrated in Figure 3, may be used to allow the high-frequency monitoring signals to be superimposed on the RF ablation waveform while utilizing only two insulated conductors (such as conductors 114, 116 shown in Figure 2). This may minimize the complexity and the profile of the modulation system 100. However, the circuit 200 illustrated in Figure 3 is merely exemplary and is not intended to be limiting.

The electrical field 124 may extend a distance into the adjacent tissue 106. In general, the resistance of the surrounding tissue 106 may decrease as the temperature of the tissue increases until a point where the tissue begins to denature or irreversibly change, for example, at approximately 50-60°C. Once the tissue has begun to denature the resistance of the tissue may increase. As the target tissue 106 is ablated, the resulting high-frequency current and phase angle 124 may be measured so that tissue variations can be detected as impedance variations.

The split electrode plates 112a,b may be sized and positioned such that the electrode plates 112a,b function analogous to a capacitor. For example, the separation distance to thickness aspect ratio may affect the shape of the electrical field 124 near the gap 126 between the first electrode plate 112a and the second electrode plate 112b. For example, by choosing a relatively narrow gap 126, the fringes of the current 124 may "reach" outward and extend into the adjacent tissue 106 far enough to measure impedance of the target tissue 106. The aspect ratio may be manipulated such that the effective region (region capable of being measured) can be chosen to be somewhat closer or somewhat farther from the split plate electrode 112a,b to monitor the blood vessel wall 104 or the target tissue 106, as desired. It is contemplated that, in some instances, the gap 126 may be approximately 0.5 millimeters and the thickness of the electrodes 112a,b may be very thin. However, it is contemplated that the gap 126 may be larger or smaller than 0.5 millimeters. For example, the gap 126 may be 1.0 millimeters or more. In some instances, the gap 126 may be smaller than 0.5 millimeters. In some instances, the thickness of the ablation electrodes 112a,b may approach zero by forming the electrodes 112a,b with a plating method or an insulating mask. It is further contemplated that the structure of the split electrodes 112a,b may be chosen to produce capacitance and the geometry to optimize impedance monitoring as desired. For example, a higher capacitance may facilitate accurate measurement of the phase angle and lower capacitance may exaggerate the fringe for deeper tissue monitoring.

It is further contemplated that the split plate electrodes 112a,b can be used to detect the location of the elongate shaft 108 within the vessel lumen 102. For example, the split plate electrodes 112a,b may be used to determine if the electrodes 112a,b are near the vessel wall 104 or are more centrally located in the vessel lumen 102 (e.g. in the blood flow). The split plate electrodes 112a,b may also be used to determine the properties of the surrounding tissue 106 or vessel wall 104. For example, the split plate electrodes 112a,b may be used to determine whether there is significant fat, plaque, and/or muscle and/or whether the local tissue 106 has changed to indicate sufficient ablation. Tissue impedance may change with ablation as proteins are thermally modified, with local perfusion, or with fluid content changes resulting from the ablation. It is further contemplated that the split plate electrodes 112a,b may be operated over a range of frequencies to determine one or more impedance values. This may better detect tissue characteristics or changes. In some instances, body impedance between the split plate electrodes 112a,b and the skin-contact ground pad 120 may also be measured.

Once the modulation system 100 has been advanced to the treatment region, energy may be supplied to the split ablation electrodes 112a,b. The amount of energy delivered to the ablation electrodes 112a,b may be determined by the desired treatment as well as the feedback obtained from the electrodes 112a,b using the high frequency monitoring signals. The modulation system 100 may also heat the surrounding tissue 106. The tissue 106 may begin to heat before denaturation of the tissue 106 occurs. It is contemplated that since impedance decreases with increasing temperatures, impedance may be used to monitor temperature effects on the surrounding tissue 106. As discussed above, once the target tissue 106 has begun to denature the resistance of the tissue may increase. The target tissue 106 nearest the ablation electrodes 112a,b may receive more energy tissue positioned further away from the ablation electrodes 112a,b and thus may begin to denature more quickly. As the target tissue 106 is ablated, the change in impedance in the tissue 106 may be analyzed to determine how much tissue has been ablated and/or the degree of denaturing. The power level and duration of the ablation may be adjusted accordingly based on the impedance of the tissue. For example, more energy may result in a larger, deeper lesion.

The modulation system 100 may be advanced through the vasculature in any manner known in the art. For example, system 100 may include a guidewire lumen to allow the system 100 to be advanced over a previously located guidewire. In some embodiments, the modulation system 100 may be advanced, or partially advanced, within a guide sheath such as the sheath 16 shown in Figure 1. Once the split plate ablation electrodes 112a,b of the modulation system 100 have been placed adjacent to the desired treatment area, positioning mechanisms may be deployed, if so provided. While not explicitly shown, the split plate electrodes 112a,b may be connected to a single control unit or to separate control units (such as control unit 18 in Figure 1) by electrical conductors 114, 116. Once the modulation system 100 has been advanced to the treatment region, energy may be supplied to the split plate electrodes 112a,b. The energy may be supplied to both the split plate electrodes 112a,b such that a high frequency monitoring signal is superimposed over the RF ablation waveform. In other embodiments, ablation and monitoring may be performed separately, using a time-sharing duty cycle. In other instances, the monitoring may only be performed when requested by the user. When using current-controlled ablation, the monitoring may be achieved by applying a voltage signal and while remaining unaffected by the ablation energy. The amount of energy delivered to the split plate electrodes 112a,b may be determined by the desired treatment as well as the feedback provided by the split plate electrodes 112a,b. It is contemplated that the elongate shaft 108 may further include an infusion lumen configured to perfuse the vessel lumen 102 with saline or other conductive fluid during the ablation procedure. In some instances, the perfused fluid may be provided at room temperature or cooler.

It is contemplated if split plate electrodes 112a,b are provided that do not extend around the entire circumference of the elongate shaft 108, the elongate shaft 108 may need to be circumferentially repositioned and energy may once again be delivered to the split plate electrodes 112a,b to adequately ablate the target tissue, if circumferential ablation is desired. The number of times the elongate shaft 108 is rotated at a given longitudinal location may be determined by the number and size of the ablation electrode(s) 112a,b on the elongate shaft 108. Once a particular location has been ablated, it may be desirable to perform further ablation procedures at different longitudinal locations. Once the elongate shaft 108 has been longitudinally repositioned, energy may once again be delivered to the ablation electrodes 112a,b. If necessary, the elongate shaft 108 may be circumferentially repositioned at each longitudinal location. This process may be repeated at any number of longitudinal locations desired. It is contemplated that in some embodiments, the system 100 may include any number of split plate electrodes 112a,b at various positions along the length of the modulation system 100 such that a larger region may be treated without longitudinal displacement of the elongate shaft 108.

In some embodiments, the elongate shaft 108 may include push and/or pull wires to deflect a distal end region 110 of the elongate shaft 108 to a desired position within the vessel lumen 102. For example, a push and/or pull wire may be attached adjacent to the distal end region 110 of the elongate shaft 108 and then extend along an outer surface of the elongate shaft 108 or along an interior passageway formed in the shaft 108 to a position where it is accessible to a user. In other embodiments, the elongate shaft 108 may incorporate a planar deflection mechanism, such as a rib and spine mechanism. However, it is contemplated that the elongate shaft 108 may be deflected in any desired manner.

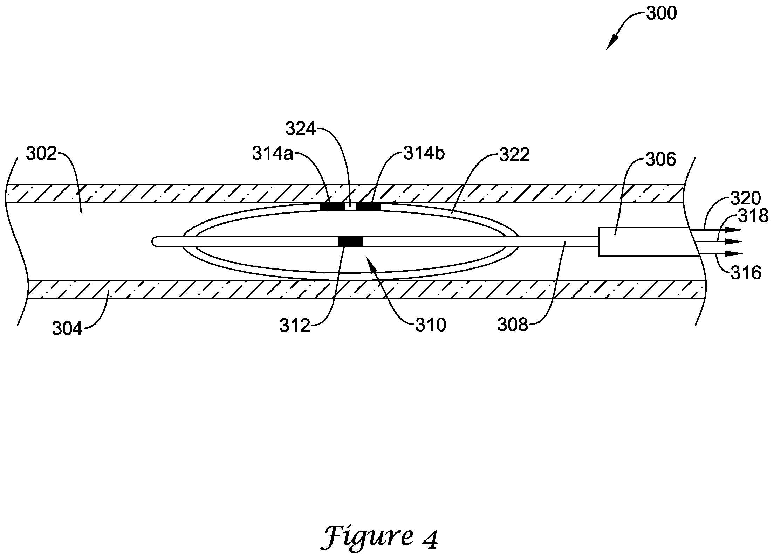

While Figure 2 illustrates the split plate electrodes 112a,b in an on-the-wall configuration, is contemplated that in some instances, it may be desirable to include an off-the wall ablation electrode which does not contact the vessel wall. Figure 4 illustrates another illustrative modulation system 300 that may be similar in form and function to other systems disclose herein. The modulation system 300 may be disposed within a body lumen 302 having a vessel wall 304. While not explicitly shown, the vessel wall may be surrounded by surrounding tissue, such as, but not limited to adventitia and connective tissues, nerves, fat, fluid, etc. in addition to the muscular vessel wall 304. It may be desirable to determine local tissue impedance and monitor tissue changes in order to control the energy delivery for proper target tissue ablation. The nerve modulation system 300 may include a split plate monitoring electrode 314a,b to determine local impedance in addition to an ablation electrode. It is contemplated that tissue impedance may be monitored during RF, ultrasound, laser, microwave, or other ablation methods.

The system 300 may include an elongate shaft 308 having a distal end region 310. The elongate shaft 308 may extend proximally from the distal end region 310 to a proximal end configured to remain outside of a patient's body. The proximal end of the elongate shaft 308 may include a hub attached thereto for connecting other treatment devices or providing a port for facilitating other treatments. It is contemplated that the stiffness of the elongate shaft 308 may be modified to form modulation system 300 for use in various vessel diameters. The elongate shaft 308 may further include one or more lumens extending therethrough. For example, the elongate shaft 308 may include a guide wire lumen and/or one or more auxiliary lumens. The lumens may be configured in any suitable way such as those ways commonly used for medical devices. While not explicitly shown, the modulation system 300 may further include temperature sensors/wires, an infusion lumen, radiopaque marker bands, fixed guidewire tip, external sheath and/or other components to facilitate the use and advancement of the system 300 within the vasculature. In some instances, the modulation system 300 may include an outer sheath 306 to facilitate advancement of the system 300 within the vasculature.

The system 300 may further include one or more ablation electrodes 312 disposed on the outer surface of the elongate shaft 308. While the system 300 is illustrated as including one ablation electrode 312, it is contemplated that the modulation system 300 may include any number of ablation electrodes 312 desired, such as, but not limited to, two, three, four, or more. If multiple ablation electrodes 312 are provided, the ablation electrodes 312 may be longitudinally and/or radially and/or circumferentially spaced as desired. The ablation electrode 312 may include similar features and may function in a similar manner to the ablation aspect of the split plate electrode 112a,b discussed with respect to Figure 2. In some embodiments, the ablation electrode 312 may be positioned adjacent to the distal end region 310 of the elongate shaft 308. In other embodiments, the ablation electrode 312 may be positioned proximal of the distal end region 310. The ablation electrode 312 may be energized at a first frequency (for example, approximately 500 kHz) to impart tissue change in the surrounding local tissue. The ablation electrode 312 may be connected through an insulated conductor 316 to a control unit (such as control unit 18 in Figure 1).

While not explicitly shown, a skin-contact ground pad may also be connected through an electrical conductor to a control unit. In operation, RF ablation energy (such as a voltage waveform) may be applied to the ablation electrode 312 such that ablation current passes through the local target tissue and additional body tissue to the ground pad. The current may heat the target tissue to provide the desired treatment. It is contemplated that in some instances, current control may be used in addition to or in place of voltage control to control the ablation.

The modulation system 300 may further include split plate electrodes 314a,b. It is contemplated that the modulation system 300 may include additional split plate electrodes 314a,b to further refine the tissue evaluation. The split plate electrodes 314a,b may include similar features and may function in a similar manner to the monitoring aspect of the split plate electrodes 112a,b discussed with respect Figure 2. It is contemplated that when an additional ablation electrode 312 is present, the split plate electrodes 314a,b may function as only a monitoring electrode. However, this is not required. It is contemplated that the split plate electrodes 314a,b may also use high frequency monitoring signals superimposed over a radiofrequency ablation waveform to provide additional tissue ablation. The split plate electrodes 314a,b may be energized at a second frequency (for example, approximately 10 MHz) to monitor and detect tissue changes in the local tissue. The split plate electrodes 314a,b may be connected through separate insulated conductors 318, 320 to a control unit (such as control unit 18 in Figure 1). It is contemplated that a choke circuit may be used to exclude interference from the high-power ablation waveform. However, the ablation electrode 312 and the split plate electrodes 314a,b may each require a separate insulated conductor 316, 318, 320 for supplying power to the electrodes 312, 314a,b.

The high frequency signal provided to the split plate electrodes 314a,b may result in a small high-frequency current between the split electrode plates 112a,b. The current may extend a distance into the adjacent tissue. In general, the resistance of the surrounding tissue may decrease as the temperature of the tissue increases until a point where the tissue begins to denature or irreversibly change, for example, at approximately 50-60°C. Once the tissue has begun to denature the resistance of the tissue may increase. As the target tissue is ablated, the resulting high-frequency current and phase angle may be measured so that tissue variations can be detected as impedance variations.

The split electrode plates 314a,b may be sized and positioned such that the electrode plates 314a,b function analogous to a capacitor. For example, the separation distance to thickness aspect ratio may affect the shape of the electrical field near the gap 324 between the first electrode plate 314a and the second electrode plate 314b. For example, by choosing a relatively narrow gap 324, the fringes of the current may "reach" outward and extend into the adjacent tissue far enough to measure impedance of the target tissue. The aspect ratio may be manipulated such that the effective region (region capable of being measured) can be chosen to be somewhat closer or somewhat farther from the split plate electrode 314a,b to monitor the blood vessel wall 304 or the target tissue, as desired. It is contemplated that, in some instances, the gap 324 may be approximately 0.5 millimeters and the thickness of the electrodes 314a,b may be very thin. However, it is contemplated that the gap 324 may be larger or smaller than 0.5 millimeters. For example, the gap 324 may be 1.0 millimeters or more. In some instances, the gap 324 may be smaller than 0.5 millimeters. In some instances, the thickness of the ablation electrodes 314a,b may approach zero by forming the electrodes 314a,b with a plating method or an insulating mask. It is further contemplated that the structure of the split electrodes 314a,b may be chosen to produce capacitance and the geometry to optimize impedance monitoring as desired. For example, a higher capacitance may facilitate accurate measurement of the phase angle and lower capacitance may exaggerate the fringe for deeper tissue monitoring.

It is further contemplated that the split plate electrodes 314a,b can be used to detect the location of the elongate shaft 308 within the vessel lumen 102. For example, the split plate electrodes 314a,b may be used to determine if the electrodes 314a,b are near the vessel wall 304 or are more centrally located in the vessel lumen 304 (e.g. in the blood flow). The split plate electrodes 314a,b may also be used to determine the properties of the surrounding tissue or vessel wall 304. For example, the split plate electrodes 314a,b may be used to determine whether there is significant fat, plaque, and/or muscle and/or whether the local tissue has changed to indicate sufficient ablation. Tissue impedance may change with ablation as proteins are thermally modified, with local perfusion, or with fluid content changes resulting from the ablation. It is further contemplated that the split plate electrodes 314a,b may be operated over a range of frequencies to determine one or more impedance values. This may better detect tissue characteristics or changes. In some instances, body impedance between the split plate electrodes 314a,b and the skin-contact ground pad may also be measured.

The modulation system 300 may further include a structure configured to place the split plate electrodes 314a,b in contact with the vessel wall 304 and maintain the ablation electrode 312 in an off-the-wall configuration. For example, in some instances the elongate shaft 308 may further include a positioning basket 322 configured to expand and engage the vessel wall 304 to center the ablation electrode 312 and bring the split plate electrodes 314a,b into contact with the vessel wall 304. In other embodiments, the elongate shaft 308 may further include a partially occlusive balloon which may be used to position the ablation electrode 312 and/or to increase the blood velocity near the ablation electrode 312 to provide better vessel wall cooling. It is contemplated that the split plate electrodes 314a,b may be formed on or otherwise secured to an outer surface of the balloon such that they are in contact with the vessel lumen. It is contemplated that the elongate shaft 308 may further include an infusion lumen configured to perfuse the vessel lumen 302 with saline or other conductive fluid during the ablation procedure. In some instances, the perfused fluid may be provided at room temperature or cooler.

The modulation system 300 may be advanced through the vasculature in any manner known in the art. For example, system 300 may include a guidewire lumen to allow the system 300 to be advanced over a previously located guidewire. In some embodiments, the modulation system 300 may be advanced, or partially advanced, within a guide sheath 306. Once the distal end region 310 has been positioned adjacent to the desired treatment region, the guide sheath 306 may be retracted proximally to expose the distal end region 310 of the elongate shaft 308. The positioning basket 322 may be configured to expand upon retraction of the guide sheath 306 or may be manually actuated by a user. The positioning basket 322 may expand to engage the vessel wall 304 such that the split plate electrodes 314a,b are in contact with the vessel wall 304. However, in other instances, the positioning basket 322 may be structured such that the split plate electrodes 314a,b do not contact the vessel wall 304.

While not explicitly shown, the ablation electrode 312 and the split plate electrodes 314a,b may be connected to a single control unit or to separate control units (such as control unit 18 in Figure 1) by electrical conductors 316, 318, 320. Once the modulation system 300 has been advanced to the treatment region, energy may be supplied to the ablation electrode 312 and the split plate electrodes 314a,b. In some embodiments, energy may be supplied to both the ablation electrode 312 and the split plate electrodes 314a,b simultaneously such that ablation and monitoring are performed at the same time. In other embodiments, ablation and monitoring may be performed separately, using a time-sharing duty cycle. In other instances, the monitoring may be performed only when requested by the user. The amount of energy delivered to the ablation electrode 312 may be determined by the desired treatment as well as the feedback provided by the split plate electrodes 314a,b. It is contemplated that the elongate shaft 308 may further include an infusion lumen configured to perfuse the vessel lumen 302 with saline or other conductive fluid during the ablation procedure. In some instances, the perfused fluid may be provided at room temperature or cooler.

It is contemplated if an ablation electrode 312 is provided that does not extend around the entire circumference of the elongate shaft 308, the elongate shaft 308 may need to be circumferentially repositioned and energy may once again be delivered to the ablation electrode 312 (and/or split plate electrodes 314a,b) to adequately ablate the target tissue, if circumferential ablation is desired. The number of times the elongate shaft 308 is rotated at a given longitudinal location may be determined by the number and size of the ablation electrode(s) 312 on the elongate shaft 308. Once a particular location has been ablated, it may be desirable to perform further ablation procedures at different longitudinal locations. Once the elongate shaft 308 has been longitudinally repositioned, energy may once again be delivered to the ablation electrodes 312 (and/or split plate electrodes 314a,b). If necessary, the elongate shaft 308 may be circumferentially repositioned at each longitudinal location. This process may be repeated at any number of longitudinal locations desired. It is contemplated that in some embodiments, the system 300 may include any number of ablation electrodes 312 and/or split plate electrodes 314a,b at various positions along the length of the modulation system 300 such that a larger region may be treated and/or monitored without longitudinal displacement of the elongate shaft 308.

In some embodiments, the elongate shaft 308 may include push and/or pull wires to deflect a distal end region 310 of the elongate shaft 308 to a desired position within the vessel lumen 302. For example, a push and/or pull wire may be attached adjacent to the distal end 310 of the elongate shaft 308 and then extend along an outer surface of the elongate shaft 308 or along an interior passageway formed in the shaft 308 to a position where it is accessible to a user. In other embodiments, the elongate shaft 308 may incorporate a planar deflection mechanism, such as a rib and spine mechanism. However, it is contemplated that the elongate shaft 308 may be deflected in any desired manner.

Those skilled in the art will recognize that the present invention may be manifested in a variety of forms other than the specific embodiments described and contemplated herein. Accordingly, departure in form and detail may be made without departing from the scope and of the present invention as described in the appended claims.

## Claims

1. An intravascular nerve modulation system (100; 300) comprising:
an elongate shaft (108; 308) having a proximal end region and a distal end region (110; 310);
a first nerve modulation element (112a; 314a) disposed adjacent the distal end region (110; 310);
a second nerve modulation element (112b; 314b) disposed adjacent to the first nerve modulation element (112a; 314a); and
a control unit (18) configured to control supplying electrical energy to the modulation elements and sensing with the modulation elements;
wherein the first and second nerve modulation elements (112a, 112b; 314a, 314b) are configured to monitor impedance of a surrounding region and ablate the surrounding region so as to function as both an ablation electrode and a sensing electrode.

2. The system of claim 1, wherein the first and second nerve modulation elements (112a, 112b; 314a, 314b) comprise a split plate electrode.

3. The system of any one of claims 1-2, wherein the first nerve modulation element (112a; 314a) is positioned approximately 0.5 millimeters to 1.0 millimeters from the second nerve modulation element (112b; 314b).

4. The system of any one of claims 1-3, wherein the first and second nerve modulation elements (112a, 112b; 314a, 314b) are plated on the elongate shaft (108; 308).

5. The system of any one of claims 1-4, wherein the control unit (18) is electrically connected to the first and second nerve modulation elements (112a, 112b; 314a, 314b).

6. The system of claim 5, wherein the first nerve modulation element (112a; 314a) and the second nerve modulation element (112b; 314b) are electrically connected to the control unit (18) by separate insulated electrical conductors (114, 116; 318, 320).

7. The system of claim 5, wherein the control unit (18) supplies energy at a first frequency to perform ablation and a second frequency to monitor a progress of the ablation.

8. The system (100; 300) of claim 1, wherein
the modulation elements comprise a split plate electrode disposed adjacent to the distal end region (110; 310) of the elongate shaft (108; 308), the split plate electrode including a first electrode plate (112a; 314a) and a second electrode plate (112b; 314b) spaced a distance from the first electrode plate (112a; 314a),
the system further comprising a ground pad (120),
wherein the first electrode plate (112a; 314a), the second electrode plate (112b; 314b), and the ground pad (120) are electrically connected to the control unit (18).

9. The system of claim 8, wherein the control unit (18) supplies energy at a first frequency to modulate a target tissue.

10. The system of claim 9, wherein the control unit (18) supplies energy at a second frequency to monitor tissue properties of the target tissue.

11. The system of claim 10, wherein the second frequency is higher than the first frequency.

12. The system of claim 11, wherein the second frequency is superimposed on the first frequency.

13. The system of any one of claims 8-12, further comprising a positioning basket secured to the distal end region (310) of the elongate shaft (308).

14. The system of claim 13, wherein the split plate electrode (314a, 314b) is positioned on the positioning basket.

15. The system of any one of claims 8-14, further comprising an ablation electrode (312) positioned on the elongate shaft (308) adjacent to the distal end region (310).

## Patentansprüche

1. Intravaskuläres Neuromodulationssystem (100; 300), aufweisend:
einen länglichen Schaft (108; 308) mit einem proximalen Endbereich und einem distalen Endbereich (110; 310);
ein erstes Neuromodulationselement (112a; 314a), das benachbart des distalen Endbereichs (110; 310) angeordnet ist;
ein zweites Neuromodulationselement (112b; 314b), das benachbart zum ersten Neuromodulationselement (112a; 314a) angeordnet ist; und
eine Steuerungseinheit (18), die zur Zufuhr von elektrischer Energie an die Modulationselemente und zum Abtasten mit den Modulationselementen eingerichtet ist;
wobei das erste und zweite Neuromodulationselement (112a, 112b; 314a, 314b) eingerichtet sind, Impedanz eines Umgebungsbereichs zu überwachen und den Umgebungsbereich zu ablatieren, wobei sie sowohl als Ablationselektrode als auch als Abtast-Elektrode fungieren.

2. System nach Anspruch 1, wobei die ersten und zweiten Neuromodulationselemente (112a, 112b; 314a, 314b) eine Elektrode mit geteilter Platte aufweisen.

3. System nach einem der Ansprüche 1 bis 2, wobei das erste Neuromodulationselement (112a; 314a) ungefähr 0,5 Millimeter bis 1,0 Millimeter vom zweiten Neuromodulationselement (112b; 314b) entfernt positioniert ist.

4. System nach einem der Ansprüche 1 bis 3, wobei das erste und zweite Neuromodulationselement (112a, 112b; 314a, 314b) auf dem länglichen Schaft (108; 308) plattiert sind.

5. System nach einem der Ansprüche 1 bis 4, wobei die Steuerungseinheit (18) mit dem ersten und zweiten Neuromodulationselement (112a, 112b; 314a, 314b) elektrisch verbunden sind.

6. System nach Anspruch 5, wobei das erste Neuromodulationselement (112a; 314a) und das zweite Neuromodulationselement (112b; 314b) elektrisch mit der Steuerungseinheit (18) durch getrennte, isolierte elektrische Leiter (114, 116; 318, 320) verbunden sind.

7. System nach Anspruch 5, wobei die Steuerungseinheit (18) Energie mit einer ersten Frequenz zum Durchführen der Ablation und einer zweiten Frequenz zum Überwachen eines Fortschreitens der Ablation zuführt.

8. System (100; 300) nach Anspruch 1, wobei
die Modulationselemente eine Elektrode mit geteilter Platte aufweisen, die benachbart des distalen Endbereichs (110; 310) des länglichen Schafts (108; 308) angeordnet ist, wobei die Elektrode mit geteilter Platte eine erste Elektrodenplatte (112a; 314a) und eine zweite Elektrodenplatte (112b; 314b) aufweist, die zur ersten Elektrodenplatte (112a; 314a) um eine Entfernung beabstandet ist,
wobei das System ferner ein Masse-Pad (120) aufweist,
wobei die erste Elektrodenplatte (112a; 314a), die zweite Elektrodenplatte (112b; 314b) und das Masse-Pad (120) elektrisch mit der Steuerungseinheit (18) verbunden sind.

9. System nach Anspruch 8, wobei die Steuerungseinheit (18) Energie mit einer ersten Frequenz zuführt, um ein Zielgewebe zu modulieren.

10. System nach Anspruch 9, wobei die Steuerungseinheit (18) Energie mit einer zweiten Frequenz zuführt, um Gewebeeigenschaften des Zielgewebes zu überwachen.

11. System nach Anspruch 10, wobei die zweite Frequenz höher ist als die erste Frequenz.

12. System nach Anspruch 11, wobei die erste Frequenz mit der zweiten Frequenz überlagert wird.

13. System nach einem der Ansprüche 8 bis 12, ferner aufweisend einen Positionierkorb, der am distalen Endbereich (310) des länglichen Schafts (308) angebracht ist.

14. System nach Anspruch 13, wobei die Platte mit geteilter Elektrode (314a, 314b) an dem Positionierkorb positioniert ist.

15. System nach einem der Ansprüche 8-14, ferner aufweisend eine Ablationselektrode (312), die an dem länglichen Schaft (308) angrenzend an den distalen Endbereich (310) positioniert ist.

## Revendications

1. Système de modulation des nerfs intravasculaire (100; 300) comprenant :
une tige allongée (108; 308) ayant une région d'extrémité proximale et une région d'extrémité distale (110; 310);
un premier élément de modulation des nerfs (112a; 314a) disposé en position adjacente à la région d'extrémité distale (110; 310);
un second élément de modulation des nerfs (112b; 314b) disposé en position adjacente au premier élément de modulation des nerfs (112a, 314a) ; et
une unité de commande (18) configurée pour commander la fourniture d'énergie électrique aux éléments de modulation et la détection avec les éléments de modulation ;
où les premier et second éléments de modulation des nerfs (112a, 112b; 314a, 314b) sont configurés pour surveiller l'impédance d'une région environnante et effectuer l'ablation de la région environnante de manière à fonctionner à la fois comme une électrode d'ablation et une électrode de détection.

2. Système selon la revendication 1, où les premier et second éléments de modulation des nerfs (112a, 112b; 314a, 314b) comprennent une électrode à plaques séparées.

3. Système selon l'une quelconque des revendications 1-2, où le premier élément de modulation des nerfs (112a; 314a) est positionné à environ 0,5 millimètre à 1,0 millimètre du second élément de modulation des nerfs (112b, 314b).

4. Système selon l'une quelconque des revendications 1-3, où les premier et second éléments de modulation des nerfs (112a, 112b; 314a, 314b) sont plaqués sur la tige allongée (108; 308).

5. Système selon l'une quelconque des revendications 1-4, où l'unité de commande (18) est reliée électriquement aux premier et second éléments de modulation des nerfs (112a, 112b; 314a, 314b).

6. Système selon la revendication 5, où le premier élément de modulation des nerfs (112a; 314a) et le second élément de modulation des nerfs (112b; 314b) sont reliés électriquement à l'unité de commande (18) par des conducteurs électriques isolés séparés (114, 116; 318, 320).

7. Système selon la revendication 5, où l'unité de commande (18) fournit de l'énergie à une première fréquence pour effectuer l'ablation et à une seconde fréquence pour surveiller une évolution de l'ablation.

8. Système (100; 300) selon la revendication 1, où
les éléments de modulation comprennent une électrode à plaques séparées disposée en position adjacente à la région d'extrémité distale (110; 310) de la tige allongée (108; 308), l'électrode à plaques séparées incluant une première plaque d'électrode (112a; 314a) et une seconde plaque d'électrode (112b; 314b) espacée d'une distance de la première plaque d'électrode (112a; 314a),
le système comprenant en outre un plot de masse (120),
où la première plaque d'électrode (112a); 314a), la seconde plaque d'électrode (112b; 314b) et le plot de masse (120) sont reliés électriquement à l'unité de commande (18).

9. Système selon la revendication 8, où l'unité de commande (18) fournit de l'énergie à une première fréquence pour moduler un tissu cible.

10. Système selon la revendication 9, où l'unité de commande (18) fournit de l'énergie à une seconde fréquence pour surveiller les propriétés tissulaires du tissu cible.

11. Système selon la revendication 10, où la seconde fréquence est supérieure à la première fréquence.

12. Système selon la revendication 11, où la seconde fréquence est superposée sur la première fréquence.

13. Système selon l'une quelconque des revendications 8-12, comprenant en outre un panier de positionnement fixé à la région d'extrémité distale (310) de la tige allongée (308).

14. Système selon la revendication 13, où l'électrode à plaques séparées (314a, 314b) est positionnée sur le panier de positionnement.

15. Système selon l'une quelconque des revendications 8-14, comprenant en outre une électrode d'ablation (312) positionnée sur la tige allongée (308) en position adjacente à la région d'extrémité distale (310).
